Europäisches Patentamt

European Patent Office

Office européen des brevets

(19)

(11) Veröffentlichungsnummer: **0 189 561**
**B1**

(12)

# EUROPÄISCHE PATENTSCHRIFT

(45) Veröffentlichungstag der Patentschrift: 18.07.90

(21) Anmeldenummer: 85115486.4

(22) Anmeldetag: 05.12.85

(51) Int. Cl.⁵: **A 61 M 1/34**

(54) Hämodiafiltrationsgerät.

(30) Priorität: 07.12.84 DE 3444671

(43) Veröffentlichungstag der Anmeldung:
06.08.86 Patentblatt 86/32

(45) Bekanntmachung des Hinweises auf die
Patenterteilung:
18.07.90 Patentblatt 90/29

(84) Benannte Vertragsstaaten:
DE FR GB IT

(56) Entgegenhaltungen:
EP-A-0 042 939
EP-A-0 044 694
EP-A-0 087 171
EP-A-0 122 604
DE-A-2 838 414
DE-A-3 407 147

(73) Patentinhaber: Fresenius AG
Gluckensteinweg 5
D-6380 Bad Homburg v.d.H. (DE)

(72) Erfinder: Polaschegg, Hans-Dietrich, Dr.
Grünwiesenweg 9
D-6370 Oberursel 4 (DE)
Erfinder: Mathieu, Bernd, Dr.
Am unteren Galgenberg 19
D-6683 Spiesen (DE)

(74) Vertreter: Luderschmidt, Wolfgang, Dr. Dipl.-
Chem.
Dr. Fuchs, Dr. Luderschmidt, Dipl.-Phys. Seids,
Dr. Mehler Patentanwälte Abraham-Lincoln-
Strasse 7 Postfach 46 60
D-6200 Wiesbaden (DE)

Courier Press, Leamington Spa, England.

## Beschreibung

Die Erfindung betrifft ein Hämodiafiltrationsgerät, ein Verfahren zum Desinfizieren, Spülen, und Entlüften und ein Verfahren zum Überprüfen der Dichtheit der zwei hintereinander geschalteten Sterilfilter eines solchen Gerätes, das einen Dialysator aufweist, der durch eine Membran in zwei Kammern geteilt ist, wobei die erste Kammer in einen Dialysierflüssigkeitsweg und die zweite Kammer in einen Blutweg geschaltet ist, der Dialysierflüssigkeitsweg eine Zuleitung, die sich von einer Einrichtung zur Bereitstellung von Dialysierflüssigkeit bis zum Dialysator erstreckt und in die eine erste Bilanzkammer eingeschaltet ist, und eine Ableitung aufweist, die sich vom Dialysator zum Abfluß erstreckt und in die eine zweite Bilanzkammer eingeschaltet ist, mit einer Pumpe zum Fördern der Dialysierflüssigkeit im geschlossenen Dialysierflüssigkeitsweg, einer zwischen den Bilanzkammern im Dialysierflüssigkeitsweg vorgesehenen Ultrafiltrationseinrichtung, einer von der Zuleitung zwischen der ersten Bilanzkammer und dem Dialysator abgehenden Verbindungsleitung, die mit dem Blutweg verbunden ist und in die zwei Sterilfilter und eine Substituat-Pumpe eingeschaltet sind, sowie mit einer Tropfkammer und einer Blutpumpe im Blutweg.

Bei der Hämodiafiltration wird—ähnlich wie bei der Hämodialyse—Blut an der Membran eines Hämofilters vorbeigeleitet, wobei ein Teil des Serums durch die Membran abgezogen wird. Dieser Anteil wird durch eine sterile Substitutionsflüssigkeit ersetzt, die entweder stromauf des Dialysators (Prädilution) oder stromab des Dialysators (Postdilution) dem extrakorporalen Blutweg zugesetzt wird. Zusätzlich wird bei der Diafiltration noch die übliche Hämodialyse durchgeführt, d.h. es wird an der Membran des Hämodialysators Dialysierflüssigkeit vorbeigeleitet, so daß über die Membran hinweg ein Austausch von harnpflichtigen Substanzen erfolgen kann.

Üblicherweise werden derzeit Substituatlösungen von Herstellern zur Verfügung gestellt, die Infusionslösungen u.dgl. herstellen. Demzufolge werden relativ großvolumige Behälter, die steriles und pyrogenfreies Substituat enthalten, vom Hersteller hergestellt und anschließend zum Patienten gebracht, was hohe Kosten nach sich zieht, mit der Folge, daß sich die Hämofiltration infolge der hohen Substituatkosten nicht sonderlich gut durchsetzen konnte.

Es wurden daher bereits Versuche unternommen, die Substituatlösung on-line, d.h. bettseitig dadurch herzustellen, daß man die übliche Dialysierflüssigkeit in den sterilen und pyrogenfreien Zustand überführt und das dadurch gewonnene Substituat anschließend dem Patienten zuführt.

Aus Trans.Am.Soc.Artif.Intern.Organ (ASAIO), 1978, S.465—467, ist die on-line-Herstellung eines sterilen und pyrogenfreien Substituats bekannt, das dadurch hergestellt wird, daß man die übliche Proportionierungseinheit zur Herstellung der üblichen Dialysierflüssigkeit einsetzt. Dabei wird ein Konzentrat in einem Verhältnis von 1:34 mit Wasser vermischt, die dadurch hergestellte Dialysierflüssigkeit anschließend entgast und erwärmt und danach zwei Ultrafiltern zugefördert, die in Reihe geschaltet sind. Am Ende des zweiten Filters wird steriles und pyrogenfreies Substituat erhalten, das anschließend dem Blutweg zugeführt wird.

Bei dieser Herstellungsart befindet sich also die Förderpumpe stromauf der beiden Ultrafilter, was sich jedoch nunmehr als nicht zweckmäßig erwiesen hat. Die Förderpumpe befindet sich nämlich auf der Druckseite des ersten Filters und förder somit die stromauf der Ultrafilter in der Dialysierflüssigkeit enthaltenen Partikel und Keime unter Druck in den ersten Ultrafilter. Hierdurch wird der sogen. "closed-end-Effekt" erzielt, d.h. die unter Druck zugeförderten Partikel setzen den Ultrafilter relativ schnell zu, so daß sich diese Anordnung nicht bewährt hat.

Aus der DE—A—34 07 147 ist ein Gerät zur Aufbereitung medizinischer Infusionslösungen bekannt, bei dem eine Konzentratflüssigkeit und Reinwasser in einem Mischbehälter in einem bestimmten Verhältnis gemischt werden. Nach dem Mischen wird die erhaltene Mischung mit Hilfe einer Pumpe lediglich einem Ultrafilter zugeführt, so daß auch hier die gleichen Nachteile der vorstehend erwähnten Anordnung auftreten, d.h. es wird wiederum der sogen. "closed-end-Effekt" erzielt.

Aus ASAIO 1979, S. 404—408, ist eine weitere on-line-Substituaterzeugungseinheit bekannt, die im wesentlichen der vorstehend erläuterten Einheit entspricht, d.h. es sind ebenfalls wieder zwei Ultrafilter im Dialysierflüssigkeitsweg angeordnet. Zusätzlich ist jedoch stromab dieser beiden Filter eine Förderpumpe in den Substituatförderweg eingeschaltet, was jedoch ebenfalls nachteilig ist, das der Druckabfall über die beiden Ultrafilter hinweg zu hoch ist, mit der Folge, daß diese Pumpe im Vakuumbereich arbeitet. Dies führt dazu, daß die üblicherweise flexible Substituatleitung zusammenfällt, also eine weitere Förderung von Substituatlösung nicht mehr möglich ist. Zusätzlich wird diese Substituatlösung nochmals entgast, was aus medizinischen Gründen höchst unerwünscht ist.

Aus der EP—A—42 939 ist ein Hämofiltrationssystem bekannt, das von den oben erwähnten Einrichtungen ausgeht, mit der Maßgabe, daß nur ein Ultrafilter zur Sterilisierung der Dialysierflüssigkeit eingesetzt wird. Dieses Filter muß speziell überwacht werden, um ein auftretendes Leck sofort zu erkennen. Diese relativ komplizierte Einrichtung wird bei den vorstehend erwähnten Systemen durch die Anordnung von zwei Filtern beseitigt, was aus ökonomischen Gründen wesentlich vorteilhafter und darüber hinaus technisch noch einfacher ist.

Der Erfindung liegt daher die Aufgabe zugrunde, ein Hämodiafiltrationsgerät der eingangs erwähnten Art so fortzubilden, daß die Ultrafilter eine hohe Standzeit besitzen und der Druckabfall im Bereich der Ultrafilter nicht zu einem Vakuum führt.

Die Lösung der Aufgabe erfolgt dadurch, daß die Substituatpumpe zwischen dem erstep Sterilfilter und dem zweiten Sterilfilter angeordnet ist.

Die erfindungsgemäße Substituatpumpe befindet sich stromab des ersten Sterilfilters und stromauf des zweiten Sterilfilters, d.h. es ist auf der Unterdruckseite mit dem ersten Sterilfilter verbunden, während die Überdruckseite zum zweiten Sterilfilter hin gerichtet ist.

Demzufolge wird der oben erwähnte closed-end-Effekt mit dem erfindungsgemäßen Gerät vermieden, da die Partikel nicht mehr in die Filterporen gedrückt werden. Dies hat zur Folge, daß die Membranen des ersten Sterilfilters eine erheblich höhere Standzeit aufweisen, d.h. die Verstopfung erst nach einer erheblich längeren Zeitdauer eintritt.

Infolge der Entfernung der Partikel im ersten Sterilfilter werden höchstens noch einige Pyrogene von der Pumpe zum zweiten Sterilfilter gefördert, so daß dieses nicht mehr durch Partikel verstopft werden kann. Dieses zweite Filter hat im übrigen lediglich die Aufgabe, eine evtl. auftretendes Leck im ersten Filter aufzufangen, also die dann hindurchtretenden Partikel und Keime von dem zu behandelnden Patienten zurückzuhalten.

Insofern kann also die Substituatpumpe im Druckbereich des zweiten Sterilfilters angeordnet sein, ohne daß sich dieses zweite Sterilfilter zusetzt. Demzufolge spielt also hier der closed-end-Effekt keine Rolle mehr.

Die Zwischenschaltung der Substituatpumpe zwischen die beiden Sterilfilter hat weiterhin den Vorteil, daß die Druckbilanz zwischen den beiden Sterilfiltern ausgeglichen ist, mit der Folge, daß das vorstehend erwähnte Vakuum bei der Anordnung der Substituatpumpe stromab der beiden Sterilfilter nicht eintritt. Demzufolge kann auch die erfindungsgemäße Substituatpumpe mit der üblichen Förderrate eingesetzt werden, ohne daß zu befürchten ist, daß ein Vakuum auftritt, was zur weiteren Entgasung der Dialysierflüssigkeit führen könnte.

Demzufolge erzeugt das erfindungsgemäße Hämodiafiltrationsgerät eine sterile und pyrogenfreie Substituatlösung für die Hämofiltration, wobei weder ein closed-end-Effekt noch ein Vakuum an den beiden Sterilfiltern durch die Wirkung der Substituatpumpe auftritt.

Erfindungsgemäß sind unter dem ersten und zweiten Sterilfilter jeweils Ultrafilter mit einer hohen Trennleistung zu verstehen, d.h. diese Filter weisen eine Trenngrenze von höchstens 40.000 Dalton auf, lassen also Molekühle mit einem höheren Molekulargewicht nicht mehr durch. Insofern unterscheiden sich diese Ultrafilter von den üblichen mikroporösen Sterilfiltern, die gewöhnlich eine Porengröße von etwa 0,2 µm aufweisen und lediglich Bakterien zurückhalten können, nicht jedoch Endotoxine (Pyrogene) sowie andere Feinstpartikel abfiltrieren können.

Gemäß einer bevorzugten Ausführungsform ist das erste Sterilfilter als hochdurchlässiges Ultrafilter ausgebildet. Hierdurch soll ein hoher Fluß der Dialysierflüssigkeit durch den Filter gewährleistet werden, d.h. die Saugleistung der Substituatpumpe kann bei dem Einsatz eines derartigen Sterilfilters relativ gering gehalten werden.

Vorteilhafterweise wird eine semipermeable Membran eingesetzt, die eine Wasserdurchlässigkeit von etwa 30—600 ml/(m² h mmHg), insbesondere etwa 100—300 ml/(m² h mmHg) aufweist. Erfindungsgemäß ist es vorteilhaft, wenn dieses erste Sterilfilter eine erheblich höhere Wasserdurchlässigkeit aufweist als das zweite Sterilfilter. Dabei soll das Verhältnis der Wasserdurchlässigkeit des ersten Sterilfilters im Vergleich zum zweiten Sterilfilter in einem Bereich von 2:1 bis 6:1, insbesondere bei etwa 4:1 liegen.

Weiterhin kann die gesamte Oberfläche des ersten Sterilfilters gegenüber dem zweiten Sterilfilter geringer sein, beispielsweise etwa die Hälfte der Oberfläche des zweiten Sterilfilters betragen. Als vorteilhaft hat sich eine Oberflächengröße von etwa 1—1,5 m² erwiesen.

Ein Beispiel für einen derartigen ersten Sterilfilter stellt der von der Anmelderin vertriebene Dialysator F60 dar, der eine Wasserdurchlässigkeit von etwa 210 ml/hm²×mm Hg und eine Membranoberfläche von etwa 1,2 m² besitzt.

An diesem ersten Sterilfilter werden die Partikel und Keime wirksam zurückgehalten, ohne daß die Gefahr des Verstopfens besteht. Des weiteren muß aufgrund der hohen Wasserdurchlässigkeit kein sonderlich hoher Saugdruck bei der Substituatpumpe eingestellt werden.

Gegenüber dem ersten Sterilfilter kann der zweite Sterilfilter—wie vorstehend bereits erwähnt—eine geringere Wasserdurchlässigkeit aufweisen, soll jedoch aber eine größere Oberfläche besitzen. In diesem zweiten Sterilfilter sollen die den ersten Sterilfilter eventuell passierenden Pyrogene im wesentlichen durch Adsorption entfernt werden, was vorteilhafterweise durch enge Poren und eine möglichst große Austauschfläche ermöglicht wird. Daneben soll das zweite Sterilfilter noch die vorstehend erwähnte Sicherheitsfunktion bei Membranbruch des ersten Sterilfilters besitzen.

Als vorteilhaft hat sich ein zweiter Sterilfilter gezeigt, der eine Wasserdurchlässigkeit von etwa 30—90, insbesondere 50—70 ml/hm²×mm Hg aufweist und eine Oberfläche von etwa 1,5—3, insbesondere etwa 2 m² besitzt. Ein solcher Filter wird beispielsweise von der Anmelderin unter der Bezeichnung D6 vertrieben.

Für die erfindungsgemäßen Sterilfilter werden die üblichen Membranmaterialien eingesetzt, beispielsweise Cuprophan oder Polysulfon.

Des weiteren können neben den üblichen Dialysefiltern als zweite Sterilfilter auch sogenannte Tiefenfilter eingesetzt werden, die eine hohe Oberfläche besitzen, wodurch die Fähigkeit zur Adsorption von Pyrogenen ansteigt.

Die erfindungsgemäße Sterilfilteranordnung ist als redundant anzusehen, da das erste und zweite Sterilfilter sich gegenseitig überwachen und jeder erste auftretende Fehler sicher durch einen Druckabfall am im Dialysierflüssigkeitskreislauf vorgesehenen Druckmeßgerät erkannt werden kann.

Zur Sicherheit wird vorteilhafterweise zwischen den Behandlungen jeweils ein Drucktest, wie nachstehend beschrieben durchgeführt, mit dem der intakte, unverletzte Zustand der Sterilfilter überprüft werden kann.

Insofern ist also erfindungsgemäß sowohl während der Herstellung der Hämofiltrationslösung eine Überwachung des intakten Zustands der beiden Sterilfilter durch eine Druckeinrichtung im Dialysierflüssigkeitskreislauf als auch eine Überprüfung der beiden Sterilfilter zwischen den Behandlungen vorgesehen.

Somit ist also die erfindungsgemäße Anordnung aufgrund ihrer Redundanz als sicher anzusehen, so daß die erzeugten Hämofiltrationslösungen sowohl steril als auch pyrogenfrei sind.

Die Erfindung bezieht sich ferner auf Verfahren gemäß den Ansprüchen 11 und 14.

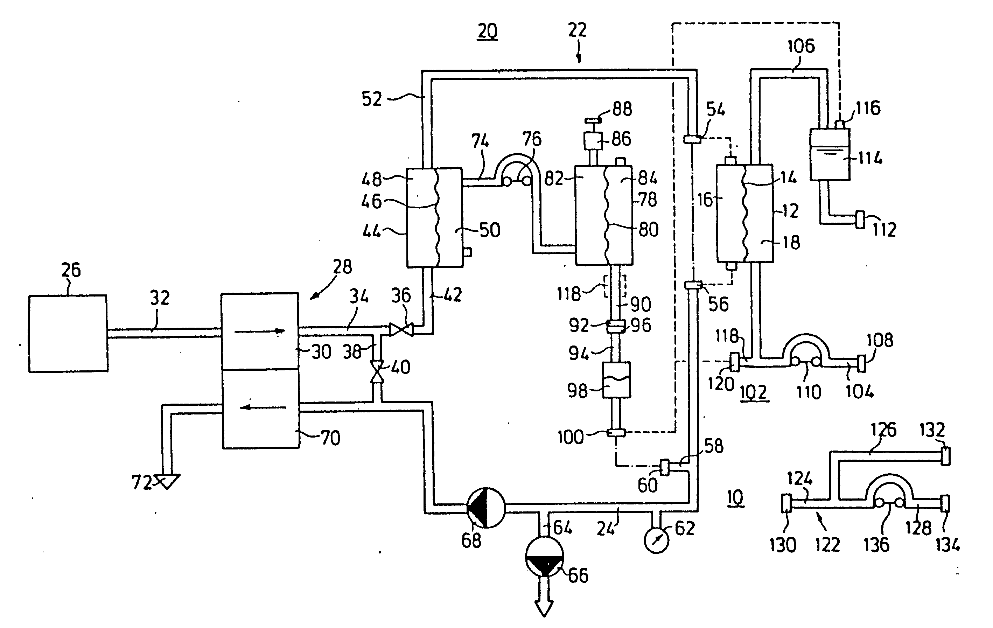

Weitere Einzelheiten, Merkmale und Vorteile der Erfindung sind anhand der nachfolgenden Beschreibung eines Ausführungsbeispiels unter Bezugnahme auf die Zeichnung erläutert.

Die Zeichnung zeigt
eine schematische Darstellung eines Hämodiafiltrationsgeräts mit zwei Sterilfiltern, zwischen denen eine Substituatpumpe angeordnet ist.

In der Figur ist mit 10 ein Hämodiafiltrationsgerät gezeigt, das einen üblichen Dialysator 12 aufweist, der durch eine Membran 14 in eine von Dialysierflüssigkeit 16 durchflossene Kammer und in eine mit Blut durchflossene Kammer 18 getrennt ist.

Die Kammer 16 ist in einen Dialysierflüssigkeitsweg 20 eingeschaltet, der aus einer Zuleitung 22 und einer Ableitung 24 besteht.

Während das eine Ende der Zuleitung 22 mit dem Eingang des Dialysators 12 verbunden ist, ist das andere Ende der Zuleitung 22 mit einer Einheit 26 zur Bereitstellung von Dialysierflüssigkeit verbunden.

Diese Einheit 26 stellt die übliche Dialysierflüssigkeit, beispielsweise auf Bicarbonatbasis, bereit.

In die Zuleitung 22 ist weiterhin eine zur Bilanziereinheit 28 gehörige Bilanzkammer 30 eingeschaltet. Diese Bilanzkammer 30 ist einerseits über das Leitungsstück 32 mit der Einheit 26 zur Bereitstellung der Dialysierflüssigkeit und andererseits über ein Leitungsstück 34 mit einem in die Zuleitung 22 eingeschalteten Dialysatorventil 36 verbunden. Stromauf des Dialysatorventils 36 geht vom dem Leitungsstück 34 eine Bypassleitung 38 ab, in die ein Bypassventil 40 eingeschaltet ist und die mit der Ableitung 24 verbunden ist.

Von dem Dialysatorventil 36 geht ein weiteres Leitungsstück 42 ab, das mit einem ersten Sterilfilter 44 verbunden ist. Dieses Sterilfilter wird durch eine Membran 46 in eine erste Kammer 48 und eine zweite Kammer 50 geteilt.

Von dem Auslaß der ersten Kammer 48 geht ein weiteres Leitungsstück 52 ab, das mit dem Eingang des Dialysators 12 mit Hilfe eines an seinem Ende befindlichen Konnektorstücks 54 verbunden werden kann. In der Zeichnung ist dieser Konnektorstück 54 im nichtverbundenen Zustand dargestellt, während der Verbindungszustand über eine gestrichelte Linie gezeigt ist.

Stromab der Kammer 16 geht die Ableitung 24 ab, die mit der Kammer 16 über ein am Ende der Ableitung 24 befindliches Konnektorteil 56 verbunden werden kann.

Beide Konnektorteile 54 und 26 können—wie mit der strich-punktierten Linie gezeigt—verbunden werden, d.h. die Zuleitung 22 und die Ableitung 24 können unter Umgehung des Dialysators 12 kurzgeschlossen werden.

Des weiteren zweigt von der Ableitung 24 eine Verbindungsleitung 58 ab, deren Ende mit einem Anschlußstück 60 versehen ist, das im nichtbelegten Zustand verschlossen ist.

Die Ableitung 24 ist weiterhin mit einem Druckmeßgerät 62, über eine abzweigende Leitung 64 mit einer Ultrafiltrationseinrichtung 66 sowie mit einer Umwälzpumpe 68 verbunden.

Schließlich ist stromab der Verbindung der Ableitung 24 mit der Bypassleitung 38 die zweite Bilanzkammer 70 des Bilanzkammersystems 28 in die Ableitung 24 eingeschaltet, an die sich der Abfluß 72 der Ableitung 24 anschließt.

Demzufolge stellt das sich von der Bilanzierungseinheit 28 erstreckende Leitungssystem, das durch den Dialysator 12 geführt ist, eine hydraulisch geschlossene Einheit dar, wie dies bereits in der DE—C—28 38 414 beschrieben ist, auf deren Ausführungen Bezug genommen wird und die zum Gegenstand dieser Beschreibung gemacht wird.

Wie bereits vorstehend erwähnt, ist in der Zuleitung 22 ein erster Sterilfilter 44 angeordnet. Von der zweiten Kammer 50 dieses Sterilfilters 44 geht eine Verbindungsleitung 74 ab, in die eine Substituatpumpe 76 eingeschaltet ist und die mit einem zweiten Sterilfilter 78 verbunden ist.

Dieses zweite Sterilfilter weist eine Membran 80 auf, die den zweiten Sterilfilter 78 in eine erste Kammer 82 und eine zweite Kammer 84 teilt. Dabei ist die erste Kammer 82 mit der Verbindungsleitung 74 verbunden. Des weiteren ist die erste Kammer 82 mit einer Ausgleichskammer 86 verbunden, deren oberes Ende mit einem hydrophoben Bakterien zurückweisenden Mikrofilter 88 verschlossen ist.

Die zweite Kammer 84 des zweiten Sterilfilters 78 weist eine Ableitung 90 auf, die an ihrem Ende ein Verbindungsstück 92 aufweist. Diese Ableitung 90 kann mit einem weiteren Leitungsstück 94 über ein komplementäres Verbindungsstück 96 verbunden werden, in das ein weiteres Mikrofilter 98 eingeschaltet ist. Dabei besitzt die in diesen Mikrofiltern vorgesehene Membran vorteilhafterweise eine Porengröße von etwa 0,2 µm. Schließlich ist das Ende des Leitungsstücks 94 mit einem Anschlußstück 100 versehen, das zum Anschlußstück 60 komplementär ist.

Dabei bilden das Leitungsstück 94, das Verbindungsstück 96, der Mikrofilter 98 und das Anschlußstück 100 einen wegwerfbaren Einmalartikel.

Die vom Blut durchflossene Kammer 18 des Dialysators 12 weist einen Blutweg 102 auf, der sich aus einer Zuleitung 104 und einer Ableitung 106 zusammensetzt. Die Zuleitung 104 weist an ihrem Ende ein Anschlußstück 108 auf, das mit dem Körper des Patienten verbunden werden kann. Des weiteren ist in die Zuleitung 104 eine Blutpumpe 110 eingeschaltet.

Die Ableitung 106 weist an ihrem Ende ebenfalls ein Anschlußstück 112 auf, das wiederum mit dem Körper des Patienten verbunden werden kann. Des weiteren können beide Anschlußstücke 108 und 112 zu einem Anschlußstück (Single-Needle) zusammengefaßt sein.

In die Ableitung 106 ist weiterhin eine venöse Tropfkammer 114 eingeschaltet, die auf ihrer Oberseite ein Anschlußstück 116 enthält, das zum Anschlußstück 100 der Substituatleitung komplementär ist.

Das Hämodiafiltrationsgerät 10 wird auf folgende Weise betrieben:

Durch die Einheit 26 wird zunächst Dialysierflüssigkeit in der üblichen Weise bereitgestellt. Die Bilanzkammer 30 fördert pro Hub ein bestimmtes Volumen Dialysierflüssigkeit in den geschlossenen Kreislauf, wobei vorausgesetzt ist, daß das Dialysatorventil 36 geöffnet und das Bypassventil 40 geschlossen sind. Die bilanzierte Dialysierflüssigkeitsmenge fließt durch das Leitungsstück 42, die erste Kammer 48 des Sterilfilters 44, das Leitungsstück 52, durch die erste Kammer 16 des Dialysators 12 sowie durch die Ableitung 24 zur zweiten Bilanzkammer 70 und von dort in den Abfluß 72. Dabei befördert die Bilanzkammer 70 das gleiche Volumen aus dem geschlossenen Kreislauf.

Zusätzlich kann die Ultrafiltrationseinrichtung 66 betrieben werden. Infolge des konstanten Volumens des Dialysierflüssigkeitskreises wird die durch die Ultrafiltrationseinrichtung 66 abgepumpte Flüssigkeitsmenge unmittelbar dem Blutkreislauf durch die Membran 14 des Dialysators 12 hindurch entnommen.

Diese Betriebsweise entspricht somit einer üblichen Hämodialyse, wie sie beispielsweise in der DE—PS—28 38 414 beschrieben ist.

Um eine Hämodiafiltration durchzuführen, ist das Anschlußstück 100 mit der venösen Tropfkammer 114 über das Anschlußstück 116 verbunden. Somit wird also eine unmittelbare Verbindung zwischen der Zuleitung 22 des Dialysierflüssigkeitskreislaufs und dem Blutweg 102 geschaffen. Diese direkte Verbindung besteht somit über die Verbindungsleitung 74, die Ableitung 90 und das Leitungsstück 94, wobei jeweils diese Leitungen mit Sterilfilter 44 bzw. 78 bzw. Mikrofilter 98 verbunden sind.

Die Substituatpumpe 76 wird nunmehr mit einer bestimmten Pumprate in Betrieb genommen. Somit wird eine bestimmte Menge Dialysierflüssigkeit aus der ersten Kammer 48 durch die Membran 46 in die zweite Kammer 50 des ersten Sterilfilters 44 gesaugt, gelangt von dort durch die Verbindungsleitung 74 in die erste Kammer 82 des zweiten Sterilfilters 78, von dort durch die Membran 80 in die zweite Kammer 84 des Sterilfilters 78 und schließlich durch die Ableitung 90, das Leitungsstück 94 und durch das in das Leitungsstück 94 eingeschaltete Mikrofilter 98 in die venöse Tropfkammer 114, wie dies durch die strichpunktierte Linie (Verbindung der Anschlußstücke 100 und 116) gezeigt ist. Es ist jedoch aber auch denkbar, daß das Leitungsstück 94 stromauf des Dialysators 12 mit dem Blutweg 102 verbunden ist, wie dies ebenfalls durch die gestrichelte Linie gezeigt ist.

Wie bereits vorstehend erläutert, stellt der Dialysierflüssigkeitskreislauf zwischen dem Bilanzierungssystem 28 und dem Dialysator 12 ein geschlossenes System dar. Durch die Wirkung der Substituatpumpe 76 wird aus diesem geschlossenen System eine bestimmte Menge Flüssigkeit entnommen, die zwangsläufig aus dem Blut durch die Membran 14 wiederum entnommen wird. Demzufolge wird also aus dem Blut des Patienten im Dialysator 12 eine bestimmte Menge Serumflüssigkeit entnommen, die stromab des Dialysators in der venösen Tropfkammer 114 durch eine äquivalente Menge Substituatlösung ersetzt wird (Diafiltration). In ähnlicher Weise kann jedoch aber auch—wie bereits vorstehend erläutert—stromauf des Dialysators eine bestimmte Menge Substituatlösung dem Blut zugesetzt werden, das im Dialysator wieder entzogen wird.

Es konnte nunmehr festgestellt werden, daß im ersten Sterilfilter die Partikel wirksam abgefangen werden können, ohne daß nach mehreren Behandlungen ein Verstopfen bzw. ein wesentlicher Leistungsabfall im ersten Sterilfilter 44 zu befürchten ist.

Des weiteren werden im zweiten Sterilfilter 78 wirksam die durch den ersten Sterilfilter 44 durchgelassenen Pyrogene abgefangen, mit der Folge, daß beim Patienten keine durch Pyrogene erzeugten Nebenwirkungen festgestellt werden konnten.

Durch die Zwischenschaltung der Substituatpumpe zwischen die beiden Sterilfilter 44 und 78 summieren sich auch nicht die Unterdrücke, mit denen die beiden Sterilfilter betrieben werden müßten. Dies führt, wie eingangs erläutert, stromab der beiden Sterilfilter durch die Summierung der Einzelunterdrücke zu einem Gesamtunterdruck, der zu einer unerwünschten weiteren Entgasung der Substituatlösung und darüber hinaus zu einem Kollabieren des Schlauchsystems führt.

Neben der Diafiltration kann mit dem erfindungsgemäßen Hämodiafiltrationsgerät eine übliche Ultrafiltration mit Hilfe der Ultrafiltrationseinrichtung 66 vorgenommen werden, so daß nicht nur das Blut mit der Substituatlösung "gewaschen" werden kann, sondern auch dem Blut eine bestimmte Ultrafiltrationsmenge entzogen werden kann.

Dieses Hämodiafiltrationsgerät 10 wird—wie nachstehend erläutert—auf folgende Weise desinfiziert, gespült, geprüft und entlüftet:

Desinfizieren

Es wird davon ausgegangen, daß zuvor dialysiert und gespült wurde; bei erstmaligem Aufbau bzw. Wechsel der Sterilfilter 44 und 78, muß mit dem Vorgang "Entlüften" begonnen werden. Hierzu wird das Anschlußstück 100 mit dem Anschlußstück 60 verbunden. Zugleich ist der Dialysierflüssigkeitskreislauf durch die Verbindung der beiden Konnektorstücke 54 und 56 kurzgeschlossen worden. Das Blutsystem selbst ist außer Betrieb. Nunmehr wird das Hämodialysegerät 10 gemäß dem Desinfektionsprogramm des infiziert, wobei die Substituatpumpe 76 mit einer bebestimmten Rate, beispielsweise etwa 200 ml/min, betrieben wird. Vorteilhäfterweise bewegt sich die Substituatpumpe 76 im Takt zum Bypassventil 40, das im Gegentakt zum Dialysatorventil 36 geschaltet ist. Demzufolge saugt die Substituatpumpe 76 von der Einheit 26 bereitgestellte Desinfektionsmittellösung von dem ersten Sterilfilter 44 in das zweite Sterilfilter 78, in dem das Flüssigkeitsniveau in der Ausgleichskammer 86 bis zum hydrophoben Mikrofilter 88 steht. Anschließend wird Desinfektionsmittellösung aus dem zweiten Sterilfilter 78 durch das Mikrofilter 98 in die Ableitung 24 des Dialysierflüssigkeitswegs 20 gedrückt. Es bleibt also bei diesem Desinfektionsschritt die Bilanzierung im geschlossenen System erhalten.

Spülen
Anschließend wird das Hämodiafiltrationsgerät 10 auf "Spülen" geschaltet, wobei die Einheit 26 nunmehr Frischwasser zur Verfügung stellt. Es wird dabei solange gespült, bis die Desinfektionsmittellösung sicher aus dem gesamten System durch Frischwasser ersetzt worden ist.

Prüfen
Um das Hämodiafiltrationsgerät 10 zu überprüfen, wird die schlauchförmige Verbindungsleitung 74 aus der Substituatpumpe 76 genommen. Ansonsten bleibt die vorstehend erwähnte Anordnung (Kurzschluß; ohne Einschalten des Dialysators) erhalten.
Um zu überprüfen, ob die beiden Sterilfilter 44 und 78 intakt sind, wird bei abgeschaltetem Bilanzkammersystem 28 die Ultrafiltrationspumpe 66 in Betrieb genommen. Da das geschlossene System nur noch über das hydrophobe mikroporöse Mikrofilter 88 mit der Umgebung in Verbindung steht, strömt Luft in die erste Kammer 82 des zweiten Sterilfilters 78 und durch die Leitung 74 in die zweite Kammer 50 des ersten Sterilfilters 44. Da die beiden Membranen 46 und 80 der beiden Sterilfilter 44 und 78 mit Wasser benetzt sind, kann die eingeströmte Luft nicht über die benetzten Filter entweichen, so daß sich hierdurch die Möglichkeit ergibt, etwaige in den Membranen 46 und 80 vorliegende Risse mit Hilfe des Unterdrucks zu überprüfen, der durch die Ultrafiltrationspumpe 66 erzeugt wird. Dieser läßt sich an dem Druckmeßgerät 62 verfolgen.
Bei intaktem Dialysierflüssigkeitskreislauf und intakten Sterilfiltern wird nach Erreichen eines Unterdrucks von etwa 520 mm Hg eine bestimmte Zeit (beispielsweise etwa 2 Min.) gewartet. Anschließend wird der Druck an dem Druckmeßgerät 62 beobachtet und die Zeit gestoppt, die der Druck braucht, um auf einen Unterdruck von etwa 500 auf 400 anzusteigen. Sollte diese Zeit größer als etwa 1 Minute sein, so werden die Sterilfilter 44 und 78 als dicht angesehen. Falls die Zeit kleiner sein sollte, so sind entweder ein oder beide Filter und/oder das Dialysierflüssigkeitssystem undicht. Dabei ist dieser Schritt praktisch identisch mit dem ersten Schritt der Druckhalteprobe zur Überprüfung der Intaktheit des Bilanziersystems.

Entlüften
Nach der Prüfstufe erfolgt die Entlüftung, d.h. die Füllung des gesamten Systems wieder mit Dialysierflüssigkeit.
Hierzu verbleibt das Anschlußstück 100 an der stromab des Dialysators 12 in der Ableitung 24 angeordneten Verbindungsleitung 58, während die Verbindungsleitung 74 wiederum in die Substituatpumpe 76 eingelegt wird. Zuleitung 22 und Ableitung 24 verbleiben im Kurzschluß.
Das Bilanzkammersystem 28 wird mit Normalprogramm betrieben und die Einheit 26 stellt wiederum Dialysierflüssigkeit zur Verfügung.
Weiterhin wird die Blutseite mit physiologischer Kochsalzlösung dadurch gefüllt, daß die beiden Anschlüsse 108 und 112 mit einem mit Kochsalzlösung gefüllten Beutel verbunden und die Blutpumpe 110 in Betrieb genommen wird. Dieser Beutel muß soviel Lösung enthalten, daß das gesamte Blutsystem damit gefüllt werden kann, wobei zusätzlich ein Vorrat von etwa einem halben Liter Kochsalzlösung im Beutel zurückbleiben soll.
Sobald Zuleitung und Ableitung 22 und 24 mit Dialysierflüssigkeit gefüllt sind, wobei die beiden Kammern 50 und 82 der Sterilfilter 44 und 78 mit Luft gefüllt bleiben, wird die Kurzschlußverbindung zwischen den Konnektorstücken 54 und 56 aufgehoben, wobei diese mit der Kammer 16 des Dialysators 12 verbunden werden. Weiterhin wird der Anschluß 100 vom Anschlußstück 60 getrennt, wobei letzteres verschlossen wird.
Nun wird der Dialysierflüssigkeitskreislauf wieder eingeschaltet und die Substituatpumpe 76 mit einer vorbestimmten Rate (etwa 100—200 ml/min) in Betrieb genommen. Das Ende des Mikrofilters 98 bzw. des Anschlußstücks 100 bleibt offen und wird in eine Position gebracht, die über der des hydrophoben Mikrofilters 88 liegt. Des weiteren ist der Anschluß 100 mit einem Auffangbehälter verbunden, da Flüssigkeit aus dem Mikrofilter 98 austreten wird.
Bei Normalbetrieb des Bilanziersystems 28 fördert die Substituatpumpe 76 zunächst die Luft aus den Kammern 50 und 82 durch das hydrophobe, mikroporöse Mikrofilter 88, wobei zugleich das hinausbeförderte Luftvolumen durch Dialysierflüssigkeit ersetzt wird, die aus der Kammer 44 entnommen wird. Infolge der Volumenkonstanz des geschlossenen Systems wird eine entsprechende Menge durch Ultrafiltration aus der Kammer 18 des Dialysators 12, d.h. unmittelbar aus dem Beutel mit Kochsalzlösung entnommen.

Nach der Entfernung der Luft aus der Kammer 50 wird die Luft ebenfalls aus der Kammer 82 durch das Mikrofilter 86 hinausgedrückt. Die zwischen der Kammer 82 und dem Sterilfilter vorgesehene Ausgleichskammer 86 soll dabei verhindern, daß der hydrophobe Mikrofilter 88 bereits durch die ersten Flüssigkeitstropfen dicht wird.

Sobald die Kammer 82 vollständig mit Dialysierflüssigkeit bez. Substituatlösung gefüllt ist, tritt Flüssigkeit aus dem Mikrofilter 98 aus. Die Substituatpumpe 76 und die Blutpumpe 110 werden dann angehalten, wenn diese austretende Flüssigkeit im wesentlichen luftfrei ist. Anschliessend wird die Verbindung zwischen den Verbindungsstücken 92 und 96 getrennt, wobei das Mikrofilter 98 verworfen und durch einen neuen Mikrofilter ersetzt wird. Anschließend wird das neue Anschlußstück 100 mit der venösen Tropfkammer 114 über das Anschlußstück 116 verbunden.

Damit dieses Verfahren funktioniert, muß bereits am Beginn der Füllphase der Blutkreislauf in Betrieb genommen sein und die Kochsalzlösung rezirkulieren.

Gegebenenfalls kann vor dem Auswechseln des Mikrofilters 98 und dem Anschluß des Anschlußstücks 100 an die venöse Tropfkammer 114 eine Probe genommen werden, um diese auf den Natriumgehalt zu überprüfen, oder aber die Leitfähigkeit bestimmt werden. Hierzu kann gegebenenfalls permanent eine Leitfähigkeitszelle, wie diese strichliert mit 118 gezeigt ist, in der Ableitung 90 vorgesehen sein.

Als Substituatpumpe 76 wird—wie vorstehend erwähnt—vorteilhafterweise eine peristaltische Schlauchpumpe eingesetzt. Andererseits kann jedoch anstelle einer derartigen Schlauchpumpe auch eine andere Pumpe, beispielsweise eine nicht voll okkludierende Zahnradpumpe eingesetzt werden. In diesem Fall ist es dann nicht mehr bei der Überprüfung notwendig, daß der Schlauch aus der Pumpe entnommen wird. Somit bleibt also sowohl der Zuführungsschlauch als auch der Abführungsschlauch mit einer derartigen Pumpe verbunden.

Wie in der Figur gezeigt, kann das Anschlußstück 100 sowohl mit dem Anschlußstück 116 der Tropfkammer 114 (post-dilution) als auch mit der Zuleitung 104 stromab der Blutpumpe 110 verbunden werden. Hierzu zweigt von der Zuleitung 104 eine Schlauchleitung 118 ab, die mit einem Anschlußstück 120 versehen ist. Dieses Anschlußstück 120 kann sowohl mit dem Verbindungsstück 92 der Ableitung 90 als auch mit dem Anschlußstück 100 in komplementärer Weise verbunden werden.

In einem solchen Fall liegt dann eine Predilution vor.

Gemäß einer weiteren Ausführungsform können sowohl die Pre-dilution als auch die Postdilution durchgeführt werden, wobei die erzeugte Substituatflüssigkeit entsprechend geteilt wird. Zu diesem Zweck ist ein y-förmiges Leitungsstück 122 vorgesehen, bei dem sich der Hauptast 124 in die beiden Seitenäste 126 und 128 aufteilt. Dabei ist jeder dieser Äste 124—128 an seinem Ende mit einem Anschlußstück 130, 132 bzw. 134 versehen.

Das Anschlußstück 130 kann entweder mit dem Verbindungsstück 92 oder mit dem Anschlußstück 100 verbunden werden, während die Anschlußstücke 132 bzw. 134 mit dem Anschlußstück 116 der Tropfkammer 114 bzw. mit dem Anschlußstück 120 der Zuleitung 104 verbunden können.

Um den erzeugten Substituatstrom sowohl für die Predilution als auch für die Post-dilution einsetzen zu können, ist zumindest eine Pumpe in einen der Seitenäste 126 oder 128 eingeschaltet. Wie in der Figur gezeigt, ist eine Pumpe 136, die vorteilhafterweise als peristaltische Pumpe ausgebildet ist, in den Pre-dilutions-Seitenzweig 128 eingeschaltet. Diese Pumpe 136 entnimmt dem Gesamtsubstituatstrom einen bestimmten Teilstrom, während der Rest durch den zweiten Seitenzweig 126 der Tropfkammer 114 zugeführt wird. Andererseits kann jedoch aber auch die Pumpe 136 in den anderen Zweig 126 eingeschaltet sein oder aber es können zwei Pumpen in beiden Seitenzweigen vorgesehen sein.

Gemäß einer weiteren Ausführungsform kann auf die Pumpe 136 verzichtet werden, wenn die Pumpe 76 als peristaltische Doppelschlauchpumpe ausgelegt ist. In diesem Fall wird einer der Seitenzweige 126 oder 128 zugleich mit der schlauchförmigen Verbindungsleitung 74 in die Schlauchpumpe 76 eingelegt, wobei sicherzustellen ist, daß aufgrund der Differenzen im Schlauchinnendurchmesser bzw. Schlauchquerschnitt das Verhältnis von Gesamtstrom zu Teilstrom bestimmt wird.

**Patentansprüche**

1. Hämodiafiltrationsgerät mit einem Dialysator (12), der durch eine Membran (14) in zwei Kammern (16, 18) geteilt ist, wobei die erste Kammer (16) in einen Dialysierflüssigkeitsweg (20) und die zweite Kammer (18) in einen Blutweg (102) geschaltet ist, der Dialysierflüssigkeitsweg (20) eine Zuleitung (22), die sich von einer Einrichtung (26) zur Bereitstellung von Dialysierflüssigkeit bis zum Dialysator (17) erstreckt und in die eine erste Bilanzkammer (30) eingeschaltet ist, und eine Ableitung (24) aufweist, die sich vom Dialysator (12) zum Abfluß (72) erstreckt und in die eine zweite Bilanzkammer (70) eingeschaltet ist, mit einer Pumpe (68) zum Fördern der Dialysierflüssigkeit im geschlossenen Dialysierflüssigkeitsweg (20), einer zwischen den Bilanzkammern (30, 70) im Dialysierflüssigkeitsweg (20) vorgesehenen Ultrafiltrationseinrichtung (66), einer von der Zuleitung (22) zwischen der ersten Bilanzkammer (30) und dem Dialysator (12) abgehenden Verbindungsleitung (74), die mit dem Blutweg (102) verbunden ist und in die zwei Sterilfilter (44, 78) und eine Substituatpumpe (76) eingeschaltet sind, sowie mit einer Tropfkammer (114) und einer Blutpumpe (110) im Blutweg (102), dadurch gekennzeichnet, daß die Substituatpumpe (76) zwischen die ersten und zweiten Sterilfilter (44; 78) eingeschaltet ist.

2. Gerät nach Anspruch 1, dadurch gekennzeichnet, daß das erste Sterilfilter (44) eine größere Wasserdurchlässigkeit aufweist als das zweite Sterilfilter (78).

3. Gerät nach Anspruch 1 oder 2, dadurch gekennzeichnet, daß das zweite Sterilfilter (78) eine größere Membranoberfläche aufweist als das erste Sterilfilter (44).

4. Gerät nach einem der Ansprüche 1—3, dadurch gekennzeichnet, daß die mit der zufließenden Substituatlösung beaufschlagte Kammer (82) des zweiten Sterilfilters (78) mit einer Ausgleichskammer (86) verbunden ist, die durch ein hydrophobes Mikrofilter (88) verschlossen ist.

5. Gerät nach einem der Ansprüche 1—4, dadurch gekennzeichnet, daß vom Auslaß der zweiten Kammer (84) des zweiten Sterilfilters (78) eine Ableitung (90) abzweigt, die an ihrem Ende ein Verbindungsstück (92) aufweist.

6. Gerät nach Anspruch 5, dadurch gekennzeichnet, daß die Ableitung (90) mit einem Leitungsstück (94) verbindbar ist, in die ein drittes Mikrofilter (98) eingeschaltet ist und die an ihrem anderen Ende ein Anschlußstück (100) aufweist.

7. Gerät nach einem der Ansprüche 1—6, dadurch gekennzeichnet, daß in der Ableitung (20) des Dialysierflüssigkeitswegs eine abzweigende Verbindungsleitung (58) vorgesehen ist, die an ihrem Ende ein Anschlußstück (60) aufweist, das mit dem Anschlußstück (100) des Leitungsstücks (94) verbindbar ist.

8. Gerät nach einem der Ansprüche 1—7, dadurch gekennzeichnet, daß die Zuleitung (22) und die Ableitung (24) des Dialysierflüssigkeitswegs (20) jeweils an ihrem Ende ein Konnektorstück (54; 56) aufweisen, die entweder miteinander unter Bildung eines Kurzschlusses oder mit der Kammer (16) des Dialysators (12) verbindbar sind.

9. Gerät nach einem der Ansprüche 1—8, dadurch gekennzeichnet, daß eine in der Ableitung (106) des Blutwegs (102) vorgesehene venöse Tropfkammer (114) ein Anschlußstück (116) aufweist, das mit dem Anschlußstück (100) des Leitungsstücks (94) verbindbar ist.

10. Gerät nach einem der Ansprüche 1—9, dadurch gekennzeichnet, daß die zweite Kammer (84) des zweiten Sterilfilters (78) über eine sich y-förmig verzweigende Leitung (122) sowohl mit der Zuleitung (104) als auch der Ableitung (106) des Blutwegs (102) verbunden ist und in einen der beiden Seitenäste (126, 128) eine Pumpe (76, 136) eingeschaltet ist.

11. Verfahren zum Desinfizieren, Spülen und Entlüften eines Hämodiafiltrationsgerätes gemäß Anspruch 1 bis 10, dadurch gekennzeichnet, daß die Ableitung (90) von der zweiten Kammer (84) des zweiten Sterilfilters (78) über den dritten Mikrofilter (98) und die Zuleitung (22) direkt mit der Ableitung (24) des Dialysierflüssigkeitsweges (20) verbunden werden, daß zum Desinfizieren von der Einrichtung (26) Desinfektionsmittellösung von der Substituatpumpe (76) mit einer bestimmten Rate von dem ersten Sterilfilter (44) in das zweite Sterilfilter (78) gesaugt wird, wobei das Flüssigkeitsniveau in der Ausgleichskammer (86) bis zum hydrophoben Mikrofilter (88) steht, und danach durch das Mikrofilter (98) in die Ableitung (24) des Dialysierflüssigkeitsweges (20) gedrückt wird, daß zum Spülen das Sterilfiltersystem (44, 78) mit von der Einrichtung (26) bereitgestelltem Frischwasser gespült wird, und daß zum Entlüften die Zuleitung (22) und die Ableitung (24) mit aus dem Einrichtung (26) bereitgestellter Dialysierflüssigkeit gefüllt werden, wobei die Kammern (50 und 82) mit Luft gefüllt bleiben, daß der Dialysator (12) zwischen Zuleitung (22) und Ableitung (24) geschaltet wird und der Ausgang des dritten Mikrofilters (98) von der Ableitung (24) getrennt wird, wobei der Ausgang des dritten Mikrofilters (98) auf einem höheren Niveau als das hydrophobe Mikrofilter (88) angeordnet wird, daß der Dialysierflüssigkeitskreislauf eingeschaltet und die Substituatpumpe (76) mit einer vorbestimmten Rate in Betrieb genommen wird, wobei der Blutweg mit einer Kochsalzlösung gefüllt wird und daß die Substituatpumpe (76) und der Dialysierflüssigkeitskreislauf außer Betrieb gesetzt werden, wenn die aus dem dritten Mikrofilter (98) austretende Flüssigkeit im wesentlichen luftfrei ist.

12. Verfahren nach Anspruch 11, dadurch gekennzeichnet, daß der Blutweg über seine Anschlüsse (108, 112) des in den Dialysator geschalteten Blutwegs (102) mit einem Kochsalz enthaltenden Behälter verbunden ist.

13. Verfahren nach den Ansprüchen 11 bis 12, dadurch gekennzeichnet, daß nachfolgend die Ableitung (90) des zweiten Sterilfilters (78) nach Abtrennung von der Ableitung (24) unter Zwischenschaltung eines Mikrofilters (98) mit der Tropfkammer (114) im Blutweg (102) verbunden wird.

14. Verfahren zum Überprüfen der Dichtheit der zwei hintereinander geschalteten Sterilfilter (44, 78) eines Hämodiafiltrationsgerätes nach Anspruch 1 bis 10, dadurch gekennzeichnet, daß die Kammern (50 und 82) der Mikrofilter (44, 78) in ungehinderte Strömungsverbindung gebracht werden und die Ableitung (90) von der zweiten Kammer (84) des zweiten Sterilfilters (78) über den dritten Mikrofilter (98) und die Zuleitung (22) direkt mit der Ableitung (24) des Dialysierflüssigkeitsweges (20) verbunden werden, daß der Dialysierflüssigkeitsweg (20) vollständig mit wässriger Lösung gefüllt wird, so daß die Membranen (46, 80) der Sterilfilter (44, 78) mit Wasser benetzt sind, daß der Dialysierflüssigkeitsweg (20) flüssigkeitsdicht gegenüber der Umgebung abgeschlossen wird, wobei die beiden Kammern (50 und 82) über das hydrophobe Mikrofilter (88) mit der Umgebung verbunden sind, daß anschließend so lange wässrige Lösung mittels der Ultrafiltrationseinrichtung (66) aus dem Dialysierflüssigkeitsweg (20) unter Füllen der beiden Kammern (50 und 82) mit Luft gepumpt wird, bis ein vorgegebener Unterdruck im Dialysierflüssigkeitsweg (20) erreicht ist, und daß der Wert des Unterdrucks nach Stoppen der Ultrafiltrationseinrichtung (66) über einen vorgegebenen Zeitraum überwacht wird.

15. Verfahren nach Anspruch 14, dadurch gekennzeichnet, daß zum Herstellen der unge-hinderten Strömungsverbindung die Verbin-dungsleitung (74) aus der Substituatpumpe (76) genommen wird.

**Revendications**

1. Dispositif d'hémodiafiltration avec un dialyseur (12) qui est divisé en deux chambres (16, 18) par une membrane (14), la première chambre (16) étant incorporée dans un circuit de liquide de dialyse (20) et la deuxième chambre (18) étant incorporée dans un circuit de sang (102), le circuit de liquide de dialyse (20) présentant une conduite d'alimentation (22), qui s'étend depuis un dispositif (26) pour l'approvisionnement en liquide de dialyse jusqu'au dialyseur (12) et dans laquelle est incorporée une première chambre de bilan (30), et une conduite d'évacuation (24) qui s'étend depuis le dialyseur (12) jusqu'à l'évacuation (72) et dans laquelle est incorporée une deuxième chambre de bilan (70), avec une pompe (68) pour faire circuler le liquide de dialyse dans le circuit fermé de liquide de dialyse (20), un dispositif d'ultrafiltration (66) prévu entre les chambres de bilan (30, 70) dans le circuit de liquide de dialyse (20), une conduite de liaison (74) qui part de la conduite d'alimentation (22) entre la première chambre de bilan (30) et le dialyseur (12) et qui est reliée au circuit de sang (102) et dans laquelle sont incorporés deux filtres stériles (44, 78) et une pompe de substituant (76), et avec une chambre compte-gouttes (114) et une pompe de sang (110) dans le circuit de sang (102), caractérisé en ce que la pompe de substituant (76) est montée entre le premier et le deuxième filtres stériles (44; 78).

2. Dispositif selon la revendication 1, caractérisé en ce que le premier filtre stérile (44) présente une plus grande perméabilité à l'eau que le deuxième filtre stérile (78).

3. Dispositif selon la revendication 1 ou 2, caractérisé en ce que le deuxième filtre stérile (78) présente une plus grande surface de membrane que le premier filtre stérile (44).

4. Dispositif selon l'une des revendications 1 à 3, caractérisé en ce que la chambre (82) du deuxième filtre stérile (78) qui est sollicitée par la solution arrivante de substituant, est reliée à une chambre de compensation (86) qui est fermée par un microfiltre hydrophobe (88).

5. Dispositif selon l'une des revendications 1 à 4, caractérisé en ce qu'une conduite d'évacuation (90), qui présente à son extrémité libre un embout de raccordement (92), part en dérivation de la sortie de la deuxième chambre (84) du deuxième filtre stérile (78).

6. Dispositif selon la revendication 5, caractérisé en ce que la conduite d'évacuation (90) peut être reliée à un tronçon de conduite (94) dans lequel est incorporé un troisième microfiltre (98), et qui présente à son autre extrémité un embout de raccordement (100).

7. Dispositif selon l'une des revendications 1 à 6, caractérisé en ce qu'il est prévu dans la conduite d'évacuation (24) du circuit de liquide de dialyse une conduite de liaison en dérivation (58) qui présente à son extrémité un embout de raccordement (60) qui peut être relié à l'embout de raccordement (100) du tronçon de conduite (94).

8. Dispositif selon l'une des revendications 1 à 7, caractérisé en ce que la conduite d'alimentation (22) et la conduite d'évacuation (24) du circuit de liquide de dialyse (20) présentent chacune à leur extrémité un connecteur (54; 56), ces connecteurs pouvant être reliés, soit l'un à l'autre en formant un court-circuit, soit à la chambre (16) du dialyseur (12).

9. Dispositif selon l'une des revendications 1 à 8, caractérisé en ce qu'une chambre compte-gouttes veineuse (114), prévue dans la conduite d'évacuation (106) du circuit de sang (102), présente un embout de raccordement (116) qui peut être relié à l'embout de raccordement (100) du tronçon de conduite (94).

10. Dispositif selon l'une des revendications 1 à 9, caractérisé en ce que la deuxième chambre (84) du deuxième filtre stérile (78) est reliée, par l'intermédiaire d'une conduite (122) bifurquant en Y, tant à la conduite d'alimentation (104) qu'à la conduite d'évacuation (106) du circuit de sang (102), et une pompe (76, 136) est montée dans une des deux branches latérales (126, 128).

11. Procédé pour désinfecter, rincer et désaérer un dispositif d'hémodiafiltration selon les revendications 1 à 10, caractérisé en ce que la conduite d'évacuation (90) de la deuxième chambre (84) du deuxième filtre stérile (78) est reliée par l'intermédiaire du troisième microfiltre (98) et de la conduite d'alimentation (22) directement à la conduite d'évacuation (24) du circuit de liquide de dialyse (20), en ce que, pour la désinfection, une solution de produit désinfectant provenant du dispositif (26) est aspirée par la pompe de substituant (76) à un débit donné du premier filtre stérile (44) dans le deuxième filtre stérile (78), le niveau de liquide dans la chambre de compensaton (86) atteignant le microfiltre hydrophobe (88), puis est refoulée à travers le microfiltre (98) dans la conduite d'évacuation (24) du circuit de liquide de dialyse (20), en ce que, pour le rinçage, le système de filtres stériles (44, 78) est rincé par de l'eau fraîche fournie par le dispositif (26), et en ce que, pour la désaération, la conduite d'alimentation (22) et la conduite d'évacuation (24) sont remplies de liquide de dialyse fourni par le dispositif (26), les chambres (50 et 82) restant remplies d'air, en ce que le dialyseur (12) est branché entre la conduite d'alimentation (22) et la conduite d'évacuation (24) et la sortie du troisième microfiltre (98) est séparée de la conduite d'évacuation (24), la sortie du troisième microfiltre (98) étant disposée à un niveau plus haut que le microfiltre hydrophobe (88), en ce que le circuit de liquide de dialyse est branché et la pompe de substituant (76) est mise en marche avec un débit prédéterminé, le circuit de sang étant rempli d'une solution de sel de cuisine, et en ce que la pompe de substituant (76) et le circuit de liquide de dialyse sont débranchés lorsque le liquide sortant du troisième microfiltre (98) est sensiblement exempt d'air.

12. Procédé selon la revendication 11, caractérisé en ce que le circuit de sang est relié, par l'intermédiaire de ses branchements (108, 112) du circuit de sang (102) branché dans le dialyseur (102), à un récipient contenant du sel de cuisine.

13. Procédé selon les revendications 11 à 12, caractérisé en ce qu'ensuite, la conduite d'évacuation (90) du deuxième filtre stérile (78), après séparation de la conduite d'évacuation (24), est reliée à la chambre compte-gouttes (114) dans le circuit de sang (102), avec intercalation d'un microfiltre (98).

14. Procédé pour contrôler l'étanchéité des deux filtres stériles (44, 78), montés l'un à la suite de l'autre, d'un dispositif d'hémodiafiltration selon les revendications 1 à 10, caractérisé en ce qu'une liaison d'écoulement non entravée est réalisée entre les chambres (50 et 82) des microfiltres (44, 78), et la conduite d'évacuation (90) de la deuxième chambre (84) du deuxième filtre stérile (78) est reliée par l'intermédiaire du troisième microfiltre (98) et la conduite d'alimentation (22) directement à la conduite d'évacuation (24) du circuit de liquide de dialyse (20), en ce que le circuit de liquide de dialyse (20) est rempli totalement de solution aqueuse, de sorte que les membranes (46, 80) des filtres stériles (44, 78) sont imprégnées d'eau, en ce que le circuit de liquide de dialyse (20) est isolé de manière étanche aux liquides vis-à-vis de l'environnement, en ce qu'on évacue ensuite par pompage du circuit de liquide de dialyse (20) une solution aqueuse, en passant par le dispositif d'ultrafiltration (66), et tout en remplissant d'air les deux chambres (50 et 82), jusqu'à l'atteinte dans le circuit de liquide de dialyse (20) d'une dépression prédéterminée, et en ce que la valeur de la dépression est surveillée pendant une durée prédéterminée après l'arrêt du dispositif d'ultrafiltration (66).

15. Procédé selon la revendication 14, caractérisé en ce que, pour réaliser la liaison d'écoulement non entravée, la conduite de liaison (74) est tirée à partir de la pompe de substituant (76).

**Claims**

1. A hemodiafiltration apparatus comprising a dialyzer (12) which is divided via a membrane (14) into two chambers (16, 18) whereby the first chamber (16) is connected to a dialysis solution path (20) and the second chamber (18) to a blood path (102), the said dialysis solution path has a supply line (22) which stretches from a means (26) of preparing dialysis solution to the dialyzer (12) and into which a first balance chamber (30) is connected and the dialysis solution path also has a discharge line (24) which stretches from the dialyzer (12) to the drain (72) and into which a second balance chamber (70) is connected, a pump (68) for transporting the dialysis solution in the closed dialysis solution cycle (20), an ultrafiltration means (66) positioned between the balance chambers (30, 70), a connecting line (74) which goes down from the supply line between the first balance chamber (30) and the dialyzer (12) and which is connected to the blood path (102) and into which two sterile filters (44, 78) and a substituate pump (76) are connected, and a drip chamber (114) and a blood pump (110) in the blood path (102) characterised in that the substituate pump (76) is connected between the first and second sterile filters (44, 78).

2. The apparatus according to claim 1 characterised in that the first sterile filter (44) has a greater water permeability than the second sterile filter (78).

3. The apparatus according to claims 1 or 2 characterised in that the second sterile filter (78) has a greater membrane surface than the first sterile filter (44).

4. The apparatus according to claims 1—3, characterised in that the chamber (82) which belongs to the second sterile filter (78) and which is subjected to the inflowing substituate solution is connected to an equalizing chamber (86) which is in turn sealed by a hydrophobic microfilter (88).

5. The apparatus according to claims 1—4, characterised in that a discharge line (90) branches off from the outlet of the second chamber (84) of the second sterile filter (78), the said discharge line (90) having a connecting piece (92) at its end.

6. The apparatus according to claim 5 characterised in that the discharge line (90) can be connected to a conduit section (94), has a third microfilter (98) connected to it and has a connecting piece (100) at its other end.

7. The apparatus according to claims 1—6, characterised in that a branching off connecting line (58) is provided in the discharge line (24) of the dialysis solution cycle (20), the said connecting line (58) having a connecting piece (60) at its end, the said connecting piece (60) being connectable to the connecting piece (100) of the conduit section (94).

8. The apparatus according to claims 1—7, characterised in that the supply line (22) and the discharge line (24) of the dialysis solution cycle both have connecting pieces (54; 56) at their respective ends which can either be connected to one another to form a short circuit or can be connected to the chamber (16) of the dialyzer (12).

9. The apparatus according to claims 1—8, characterised in that a venous drip chamber (114) provided in the discharge line (106) of the blood path (102) has a connecting piece (116) which can be connected to the connecting piece (100) of the conduit section (94).

10. The apparatus according to claims 1—9, characterised in that the second chamber (84) of the second sterile filter (78) is connected via a y-shaped branching conduit (122) both to the supply line (104) and to the discharge line (106) of the blood path (102) and a pump (76, 136) is connected into one of the two side branches (126, 128).

11. Procedure for sterilisation, flushing and venting of a hemodiafiltration apparatus according to claims 1 to 10, characterised in that the discharge line (90) from the second chamber (84) of the second sterile filter (78) via the third microfilter (98) and the supply line (22) are directly connected to the discharge line (24) of the dialysis solution path (20), and that for the sterilisation of the apparatus (26) the substitute pump (76) sucks a disinfectant solution from the first sterile filter (44) into the second sterile filter (78) at a predetermined rate and whereby the liquid level in the equalizer chamber (86) is up to the hydrophobic filter (88) and thereafter the disinfectant solution is forced out through the microfilter (98) into the discharge line (24) of the dialysis solution path (20), and that for flushing the sterile filter system (44, 78) is flushed with fresh water prepared by the apparatus (26), and that for venting the supply line (22) and the discharge line (24) are filled with dialysis solution prepared by the apparatus (26), whereby the chambers (50, 82) remain filled with air, and that the dialyzer (12) is connected in between the supply line (22) and the discharge line (24) and the outlet of the third microfilter (98) is separated from the discharge line (24), whereby the outlet of the third microfilter (98) is at a higher level than that of the hydrophobic microfilter (88), and that the dialysis solution cycle is switched on and that the substitute pump (76) is operated at a predetermined rate, whereby the blood path is filled with a saline solution and that the substitute pump (76) and the dialysis solution cycle are stopped when the liquid emerging from the third microfilter (98) is essentially free from air.

12. Procedure according to claim 11, characterised in that the blood path is connected to a container containing saline solution via the connectors (108, 112) of the blood path (102) connected into the dialyzer.

13. Procedure according to claims 11 to 12, characterised in that subsequently the discharge line (90) of the second sterile filter (78) is connected to the venous drip chamber (114) in the blood path (102) after separation from the discharge line (24) and when a microfilter (98) is incorporated.

14. Procedure to test the tightness of the two sterile filters connected in sequence (44, 78) of a hemodiafiltration apparatus according to claims 1 to 10, characterised in that there is an uninterrupted flow connection between the chambers (50 and 82) and the microfilters (44, 78) and the discharge line (90) from the second chamber (84) of the second sterile filter (78) via the third microfilter (98) and the supply line (22) are directly connected to the discharge line (24) of the dialysis solution path (20), and that the dialysis solution path (20) is completely filled with a watery solution so that the membranes (46, 80) of the sterile filters (44, 78) are sprinkled with water, and that the dialysis solution cycle (20) is sealed off tightly from its surroundings so that no liquid can escape, whereby the two chambers (50 and 82) are connected to the surroundings via the hydrophobic filter (88) and that thereafter a watery solution is pumped out of the dialysis solution path (20) by means of an ultrafiltration pump (66), the two chambers (50 and 82) being filled with air, as long as is necessary until a prescribed negative pressure in the dialysis solution path is reached, and that the value of the negative pressure is monitored for a prescribed period of time after the ultrafiltration pump (66) is stopped.

15. Procedure according to claim 14, characterised in that in order to produce the uninterrupted flow connection the connecting line (74) is taken out of the substitute pump (76).

1